Europäisches Patentamt

European Patent Office

Office européen des brevets

(19)

(11) Publication number: **0 003 173**
**A1**

## EUROPEAN PATENT APPLICATION

(12)

(21) Application number: **79300041.5**

(22) Date of filing: **10.01.79**

(51) Int. Cl.²: **A 61 K 39/00**
**C 12 K 9/00, A 61 K 39/40**
**A 61 K 39/42**
**//G01N33/16**

(30) Priority: **11.01.78 US 868604**

(43) Date of publication of application:
**25.07.79 Bulletin 79/15**

(84) Designated contracting states:
**BE CH DE FR GB IT LU NL SE**

(71) Applicant: **THE MASSACHUSETTS GENERAL HOSPITAL**
**55 Fruit Street**
**Boston Massachusetts 02114(US)**

(72) Inventor: **Black, Paul H.**
**21 Dawes Road**
**Lexington Massachusetts(US)**

(72) Inventor: **Haber, Edgar**
**83 Ridgeway Road**
**Weston Massachusetts(US)**

(72) Inventor: **Zurawski, Vincent R., Jnr**
**2 Sandspur Lane**
**North Reading Massachusetts(US)**

(74) Representative: **Blake, John Henry Francis**
**BROOKES AND MARTIN High Holborn House 52/54**
**High Holborn**
**London WC1V 6SE(GB)**

(54) Process for producing human antibodies and composition containing them.

(57) Human antibodies are produced specifically from human lymphocyte cell lines grown in vitro. The cells utilized to initiate the cell lines are obtained either in vivo by sensitizing a human host with the antigen to the antibody desired or in vitro by sensitizing human lymphocytes with the antigen and isolating the thus sensitized cells. The isolated cells are transformed with a lymphotropic virus and are grown to establish a continuous cell line capable of producing the human antibody specifically.

EP 0 003 173 A1

- 1 -

This invention relates to a process for forming antibodies and more particularly to a process for forming antibodies utilizing continuous human cell lines.

Presently, antibodies are produced routinely by injecting an antigen into an animal, bleeding the animal after a suitable incubation time and isolating the antibody from the serum. Since the antibodies thereby produced are heterologous, there is the attendant risk of anaphylaxis and other allergic reactions. Furthermore, the immunogenic antibodies cannot be used repeatedly in the same patient for diagnosis and therapy.

Prior to the present invention, continuous lymphoid cell lines from various sources including plasmacytomas and lymphomas of human origin have been established which synthesize and secrete immunoglobulin(s). Unfortunately, none of the antibody produced in this manner has a desired predetermined specificity. For example, a prior attempt to initiate human lymphoblastoid cell lines in vitro capable of synthesizing specific antibody yielded cell lines synthesizing gamma globulin which was not of the desired specificity. However, continuous cell lines synthesizing antibody with a specificity directed toward a bacterial vaccine have been established by in vitro viral infection of cultures from the spleen of a rabbit hyperimmunized with the bacterial vaccine. In addition, murine lymphoid cell lines yielding antibody of predetermined specificity have been established by fusing mouse myeloma cells with normal mouse lymphocytes from immunized animals. However, like antibodies obtained from the blood sera of non-human animals, the antibodies produced from these cell lines suffer the disadvantages associated with all heterologous antibodies.

Thus, prior to the present invention, there is no procedure available for producing in vitro human antibodies of a predetermined specificity from a continuous cell line. It would be highly desirable to produce human antibody of desired specificity in vitro from a continuous cell line. The human antibody obtained would be safer for use than heterologous antibody since the risk of anaphylaxis and other allergic reactions would be eliminated. Continuous production of human antibody would be of enormous benefit in the prophylaxis and treatment of certain human infectious diseases, particularly viral, where the effectiveness of present-day chemotherapy has not been demonstrated conclusively as well as the neutralization of effects of endogenous hormones extrinsically administered toxins or drugs or in vivo diagnosis using radioactively labeled antibodies. The vehicle for producing large amounts of the antibody in vitro would provide a source of antibody which is not antigenic and which could be utilized repeatedly in the same patient for diagnosis and therapy.

According to the present invention there is provided a process for producing a human antibody which comprises:

a.   administering the antigen for the antibody to human lymphocyte cells to stimulate production of said antibody by said cells,

b.   transforming said cells with a lymphotropic virus,

c.   growing said transformed cells in vitro,

d.   concentrating the transformed cells capable of producing said antibody specifically from other transformed cells that produce immunoglobulin without said antibody,

e. growing said concentrated transformed cells in a growth medium to form continuous cell lines, and

f. recovering said antibody from said continuous cell lines.

In accordance with this invention, human continuous cell lines are established which produce human antibodies of the desired specificity. The cell lines are produced from human lymphocyte cells which have been sensitized with the antigen to the antibody desired either in vivo or in vitro. The cells are transformed in vitro with a lymphotropic virus to render them autonomous and then are grown as continuous established cell lines. The cell lines are selected which survive and are capable of producing the human antibody specifically on a continuous basis.

The human cells utilized in the process of this invention are lymphocytes which are capable of producing antibody and which are capable of being transformed with a lymphotropic virus to form a continuous cell line in vitro. These cells are sensitized in vivo to the antibody desired by administering the antigen for the antibody to the human host in sufficient amounts to stimulate the cells to produce the antibody in vivo. Any antigen may be utilized so long as its administration does not present a health hazard to the human host. The sensitized cells then are removed from the human host and are transformed to malignant cells by a lymphotropic virus such as the human Epstein-Barr virus, or another herpes virus such as cytomegalovirus. The transformed cells then are grown in a conventional growth medium in order to establish one or more continuous cell lines that secrete the antibody specifically. Generally, in this procedure, a plurality of continuous cell lines are established, the majority of which secrete human gamma globulin while a minority of the cell lines produce the desired antibody specifically. The cell lines which produce the antibody specifically are identified by analyzing the antibodies secreted such as by conventional radioimmunoassay techniques. The cell lines producing the specific antibody may then be segregated by cloning or by limiting dilution, for example producing cells can be separated as an individual cell or diluted with lesser producing cells such as a 1:100 cell dilution. The cells are grown in a conventional culture medium. The antibody secreted is recovered by affinity chromatography procedures.

When immunizing in vitro, fresh lymphoid cells are obtained from operative specimens, e.g., hemolytic anemia, hypersplenism, tonsil cells. The cells then are dispersed as suspensions in a suitable suspension medium such as RPMI 1640 or T199 (Grand Island Biological). Each culture then is treated with the desired antigen in the suspension for an incubation period of sufficient duration to allow the cells to establish antibody production. Antibody production is usually established during a period of from five to fourteen days with peak production generally occurring at about seven days. Antibody production can be monitored by any conventional means such as by modification of the Jerne hemolytic plaque assay technique, Jerne et al, Science, 140:405 (1963). Specificity of the responses can be determined by adding soluble antigen to the assay mixture in order to inhibit the formation of plaques. Antigen concentration and culture technique, such as density of cell population and the nature of the medium can be varied with the antigen utilized in order to achieve the maximum number of plaque-forming (antibody-producing) cells. Concentration of specific plaque-forming (antibody-producing) cells can be effected in any conventional manner such as by utilizing the general technique of affinity chromatography of cells or through the use of cell sorting apparatus. While separation of the antibody-producing cells is preferred prior to transforming the cells, this step is not critical to the present invention since the antibody-producing cells can be separated subsequent to cell transformation. It is preferred to utilize cell separation steps both prior to and subsequent to cell transformation.

A useful cell separation technique comprises a variant of affinity chromatography techniques reported by Schlossman et al, J. Immunol., 110:313 (1973) which allows for recovery of about 98% of B cells. This method may be adaptable to antigen specific cells. The procedure would involve a two-stage separation. First, antibody-producing cells are absorbed to antigen conjugated to Sephadex. After removal by washing of nonabsorbed cells, the Sephadex is digested with dextranase and adherent cells then are recovered. An example of application of this method utilizing human spleen cells and digoxin antigen comprises coupling digoxin to a protein different than a coupling protein utilized during the step of sensitizing the cells to digoxin in order to concentrate only those cells specific for the haptenic determinant. The conjugate can be synthesized for example by the method described by Smith et al, Biochemistry, 9:331 (1970) and then coupled to Sephadex. Columns are packed in disposable syringes and washed with a suitable medium such as Eagles' minimal essential medium containing 5% bovine serum and 5mM EDTA. The cell suspension obtained from the in vitro antigen stimulation step then is treated to remove phagocytic cells such as by addition of iron carbonyl and then washing by centrifugation. For example, when utilizing 20 ml columns, $10^7$ lymphocytes can be passed down the column. The columns then are sealed at the bottom, and allowed to incubate with 1 mg of dextranase at 23°C. After about 1 hour's digestion, the resulting cell suspension can be spun down for enumeration and tested for plaque forming activity.

Alternatively, a fluorescence activated cell sorter can be utilized to isolate the antibody-producing cells, Bonner et al, Rev. Sci. Instru., 43:404 (1972). Fluorescein-labeled antigen, e.g. digoxin conjugate of ribonuclease can be prepared according to the method of Cebra et al, J. Immunol., 95:230 (1965). Cell suspensions then are stained with the dying agent and then are utilized in the operation of the cell sorter as disclosed by Fathman et al, J. Immunol., 115:584 (1975).

In some cases, rosetting of antibody-producing cells with antigen coated erythrocytes and preparation of these from the unrosetted lymphocyte populations may be accomplished using Ficoll-isopaque gradient centrifugation. Rosetting of B-lymphocytes with erythrocytes coated with $(Fab')_2$ fragments of anti-$(Fab')_2$ antibody has also been utilized in a method for enriching specific antibody-producing cells (Walker et al, Fed. Proc. 36:1288 (1977). Rosetting is accomplished subsequent to elimination of surface immunoglobulin from specific antibody-producing cells by exposure to antigen under conditions that allow patching and capping. Non-rosetted cells are then collected by Ficoll-isopaque gradient centrifugation. This population is enriched in B-lymphocytes producing antibody directed against the antigen to which the initial cell population was exposed.

The enriched antibody-producing cells then can be transformed with a virus such as Epstein-Barr virus (EBV). Cell transformation can be conducted either in the presence or in the absence of the antigen. The cells are suspended in a conventional cell medium and innoculated with a cell-free virus. Cell counts are obtained periodically and transformed cells can be stained for antigen, for example by the method of Reedman et al, Int. J. Cancer, 11:499 (1973).

These subcultures then are treated with labeled aminoacids and the supernatants tested for production of human immunoglobulin G, for example by a double antibody radioimmunoassay, utilizing rabbit antihuman immunoglobulin G antiserum.

Amounts of specific antibody may be determined by co-precipitation radioimmunometric analysis or by solid phase radioimmunometric methods as described in Example I. (Rosenthal et al, Appl. Microbiol. 25:567 (1973).

Should infectious virus be present in the culture fluids, differential inactivation procedures can be carried out utilizing the difference of immunoglobulin and virus such as by treatment with heat, formaldehyde or use of antivirus antiserum. Of course, the procedure set forth herein can be utilized for cells sensitized in vivo or in vitro.

The process of this invention is useful in producing a wide variety of human antibodies in a specific manner. Thus, the process of this invention provides substantial advantages over the prior art in that it is capable of producing antibodies from continuous human cell lines so that significant amounts of such antibodies can be produced which do not have the problems associated with heterologous antibodies. Purification of specific antibodies from cell supernatants utilizing non-specific or specific precipitation of immunoglobulin as well as specific antigen affinity chromatography (immunoadsorption) can be accomplished. Antibodies produced by the present invention are any and all antibodies which human lymphocyte cells are capable of producing when exposed to an antigen. Representative antibodies include surface antigenic determinants of lymphocytes such as that of the T cell, that of the B cell, that of the macrophage; antibodies specific for an antigenic determinant defining an organ, a tumor or a specific tissue. Various antiviral antibodies such as antiherpes simplex, anticytomegalovirus or the like, anti-HLA (histocompatability antigen) antibody, anti-tumor angiogenesis factor antibody, antibodies to drugs such as digoxin, barbiturates, amphetamines or the like, and antibodies to human hormones such as parathyroid hormone renin or the like. In addition, the process of this invention can be utilized to form specific antibodies which can be used to purify materials by affinity chromatography. Human antibodies may also be useful in the specific detection of organs, tissues or tumors which are characterized by unique antigens. For example myosin, specific antibody may be useful in the detection of myocardial infarcts, carcinoembryonic antigen specific antibody in the detection of colonic tumors.

In the process of this invention, a human host, who has been immunized from the antigen under consideration in the sense that his body is producing or is capable of producing the antibody desired, is administered the antigen in order to sensitize the cells in vivo to produce significant amounts of antibody. The antigen generally is administered by a parenteral route, such as intramuscularly, over a period to permit the cells to produce the antibody without causing the disease resulting from the antigen to occur in the host. Suitable antigens include inactivated tetanus toxin such as tetanus toxoid, or a purified viral component such as a glycoprotein or the like. Generally, the tetanus antigen is administered in dosages between about 0.3 ml and 0.6 ml, usually at 0.5 ml either once (in an already sensitized individual) or over a period of at least about 1 month, preferably between about one and two months.

The sensitized cells then are removed from the

- 9 -

0003173

human host such as by drawing blood from the host such as by veni-puncture. The recovered sensitized cells then are exposed to a lymphotropic virus to transform the cells to malignant cells. The virus is added to containers in which the cells are being cultured in an amount of between about $10^4$ to $10^6$ transforming doses per ml, generally at $10^5$ transforming doses per ml. Virus may be added to the cultures at various times, usually between 20 and 30 hours after initiation. After the cells have been transformed, as indicated by increased growth of the cells, as reflected by acidification of the medium, and the like, the cell cultures capable of producing the specific antibody desired are identified by analyzing the cell secretion by specific assay techniques such as by conventional radioimmunoassay techniques.

The cells identified to produce the desired antibody in a specific manner are segregated and placed in a conventional culture medium to provide the necessary nutritional composition for continuous cell growth or expanded for increased growth. The antibody produced by these cells then is recovered by conventional techniques, such as by affinity chromatography.

The following examples illustrate the present invention and are not intended to limit the same.

### EXAMPLE I

This example illustrates the method of this invention utilized to produce human antitetanus antibody.

Healthy adult human volunteers were each given one booster immunization of tetanus toxoid (0.5 ml). Blood was drawn at various time intervals subsequent to this injection beginning about 4 days after the injection. The mononuclear cell fraction consisting of lymphocytes and monocytes was isolated by the Ficoll-isopaque method Byum, J. Clin. Lab. Invest., 21, (Suppl 97), 77, (1968). Cultures of these cells and of cells similarly isolated from the blood of unboosted individuals were established in plastic 24-well tissue culture plates. Culture medium consisted of RPMI 1640 supplemented with 15% fetal calf serum, 2 mM glutamine and additional additives as described by Click et al, Cell. Immunol., $\underline{3}$, 264, (1972). Average initial cell density was 6 x $10^6$/ml. Production of antitetanus antibodies in serum and cell culture was monitored by a solid phase radioimmunometric assay, Rosenthal et al, Appl. Microbiol., $\underline{25}$, 567, (1973). Briefly, wells of Cooke soft plastic microliter plates were coated with tetanus toxoid, then with fetal calf serum. After washing the wells, samples were applied and allowed to incubate. Following another wash, $^{125}$I-labeled and purified rabbit antihuman gamma globulin or rabbit antihuman (Fab')$_2$ was applied to the wells and incubated. A third washing and drying procedure was followed by cutting and counting individual wells. Standard curves were derived by assaying dilutions of affinity column purified human antitetanus antibodies. These standard antitetanus antibody preparations obtained from human sera generally contained about 15% active antibody in the purified fractions. Appropriate correction was made in determining absolute values of antibody concentration in the samples. Counts bound were in direct proportion to the amount of antitetanus antibody present. Specificity of the assay for tetanus toxoid was determined routinely by addition of the toxoid to samples, which inhibited binding of antibodies. Also, cell media from cultures failed to give positive results in the assay. Furthermore, assays performed on plates coated only with fetal calf serum also failed to give positive results. Production of human gamma globulin by the cells was examined, using $^{125}$I-labeled staphylococcal protein A, Forsgren et al, Immunol., $\underline{97}$, 822 (1966), Kronvall et al, J. Immunol., $\underline{103}$, 828 (1969) and Kronvall et al, Immunochemistry,

$\underline{7}$, 124 (1970) as the probe in a solid phase assay similar to that described above. Cell cultures and continuous lines were generally assayed 4 - 5 days after feeding or subculture.

To some of the tissue culture wells, 0.2 ml of a pool of Epstein-Barr virus of the B95-8 strain, Miller et al, Proc. Nat. Acad. Sci. USA, $\underline{70}$, 190 (1973), was added, usually 24 hours after initiation of the culture. Virus to cell multiplicity was approximately 0.01 using a virus preparation which contained $10^5$ transforming doses per milliliter. The cells were maintained as stationary suspension cultures in a humid atmosphere of 5% $CO_2$ in air. Following infection, cultures were fed two or three times each week by removing one fourth to one half the medium and replacing it with fresh medium. Proliferating cells from individual wells were passed to succeedingly larger volumes and established as continuous lines with routine passage at 1:1 dilution in 75 or 150 $cm^2$ tissue culture flasks.

Limiting dilution cloning experiments were performed on these continuous lines at several passage levels. Serial five- to tenfold dilutions containing $10^5$ through 10 cells per milliliter were made either with fresh medium or one half conditioned and one half fresh medium. Cells were then cultured at concentrations of from $10^4$ through 1 cell per well in Costar 96-well microliter dishes without or with feeder layers of human embryonic kidney cells, human amnion cells, or human foreskin fibroblasts.

Individuals not recently·immunized (>2 yr) with tetanus toxoid usually had serum concentrations of antitetanus antibody in a range of 0.01 to 0.1 mg/ml. Booster immunization produced 10- to 100-fold increases in serum concentration of specific antibody. Most individuals achieved peak serum concentrations near 1 mg/ml of antitetanus antibody with two weeks of immunization.

Cell cultures established from Ficoll-isopaque fractionation of whole human blood of volunteers also were monitored for antitetanus antibody production. With cells from unboosted individuals, quantities of antitetanus antibody secreted into culture media were generally very small or undetectable (<1ng/ml). In cultures of cells initiated from individuals 1-4 weeks subsequent to booster immunization, antitetanus antibody secrete into culture media ranged from 0.1 µg/ml to values in excess of 1.0 µg/ml.

At the end of one month, no viable cells were detectable by trypan blue dye exclusion in cell cultures initiated from three boosted and three control individuals in 110 replicate samples to which no Epstein-Barr virus had been added. Epstein-Barr virus was added to 260 additional cultures established from 15 bleedings of six recently boosted individuals. After varying periods (3-8 weeks), significant cellular proliferation could be seen in many wells exposed to virus. Of these, 44 continuous lines (17%) were successfully subcultured. Establishment of long-term cultures was quite variable among cells derived from different persons. From 7 to 29% conversion was achieved depending on the donor. These continuously proliferating cells were chiefly lymphoblastoid in appearance. Of the 44 cultures yielding continuous lines, 7 secreted detectable antitetanus antibody in association with the burst of cell proliferation occurring from 3-8 weeks in culture. These 7 were all obtained from cultures initiated from three bleedings of two individuals. Only three cultures out of seven producing antitetanus antibody contained toxoid at the time of initiation and infection. Of 41 remaining wells containing toxoid (0.5 µg/ml), 8 continuous lines were derived that never produced any detectable antitetanus antibody. All 44 lines secreted some human gamma globulin.

An example of comparative gamma globulin and specific antibody synthesis by a group of continuous lines from one volunteer is illustrated in Table I. These lines at passage three had been in culture over two months at the time this assay was performed. Only lines 3GC-B5 and C5 contained tetanus toxoid in initial cultures. The production of gamma globulin and antitetanus antibody are not necessarily parallel. For example, whereas 3GC-B5 is secreting nearly 6 μg/ml of gamma globulin, no antitetanus antibody is detectable. In contrast, more than 10% of the gamma globulin produced by 3GC-C2 is antitetanus antibody.

Three continuous lines with relatively high initial antitetanus antibody production have been examined for extended periods of time (beyond passage 5; more than 3 months in culture). All three have shown evidence of decreasing production with extended passage. Data for two of these lines are shown in Table II. Although in each case, antitetanus antibody production is detectable even beyond passage 20 (approximately five months in culture), initial secretion of 0.1-0.3 μg/ml in 4-5 days declined to levels in the 1-2 ng/ml range. It is also clear that total gamma globulin production, as detected by the protein A assay, did not decline as rapidly. For example, for 4LP-B4 even at passage 40 (over seven months in culture), more than 0.2 μg/ml of gamma globulin is still detectable.

To determine whether or not the decrease observed in specific antibody production was due to more rapid proliferation of non-antibody secreting cells, the antitetanus antibody producing capacity of several subcultures from limiting dilution experiments was examined. From dilutions made above passage 30, utilizing human embryonic kidney cells as a feeder layer, no subcultures producing significant amounts of antitetanus antibody were found in 194 wells with growing colonies. Below passage 23 line 4LP-B4 and 3 GC-C2 with human embryonic kidney, human foreskin fibroblasts or no feeder layers, 102 out of 916 wells with growing colonies produced antitetanus antibody in excess of the parent line at the passage of dilution. Many of these subcultures of the parent lines were re-established in continuous culture. Nineteen such subcultures of cell line 3GC-C2 are shown in Table III. All of those listed in the first column of Table III were established from at least tenfold dilutions of parent cultures and all at passage 10 or beyond, i.e., past three months in culture for the parent line. Data in the fourth column denotes antibody production after two additional passages out of those wells. Subcultures 3B10, 2HB and F3 show particularly striking increases in antibody production when compared to data for the parent line shown in Table II.

A second series of subcultures at limiting dilution was carried out with several of the sublines illustrated in Table III. Production of antitetanus antibody in these subcultures was directly related to the number of initiating cells. For example, antibody production averaged $190 \pm 30$ ng/ml in 30 replicate wells initiated with $10^4$ cells of subpassage 1D2 and $31 \pm 2$ ng/ml with $10^3$ initial cells.

Progressive loss of specific antibody was observed in succeeding generations of the parent cell lines. A possible explanation is that in vitro stimulus producing de novo synthesis of antibody might occur in untransformed cells relatively early in culture. Loss of these untransformed cells with passage might then explain the decline of specific antibody synthesis observed. A candidate for such a stimulation might be macrophage bound antibody. This explanation is unlikely, however, since several subcultures in limiting dilution experiments produced antibody in excess of the parent line. By passage 23, for example, dilutions of the original media were approximately $10^{14}$. This means that subculture 3B10 (Table III) is a $10^{16}$-fold dilution of the original culture, maintained in vitro for over six months and still secreting more than 1 ng/ml of antitetanus antibody.

The explanation for progressive loss of antibody production which we regard as more likely, is that the parent lines in each case are multiclonal in nature, and that only a relatively small number of the transformed lymphocytes are producing antitetanus antibody with each succeeding generation in vitro, the antitetanus antibody producing cells, apparently not those best adapted to the tissue culture conditions, constitute less and less of the total cell population. This is consistent with the limiting dilution data. It is also consistent with the multiclonal infective potential of Epstein-Barr virus.

Clones derived from limiting dilution to the single cell level produced a constant amount of antitetanus antibody, proving that progressive loss of antibody production in heterogeneous cultures was consequent to overgrowth of non-antibody producing cells.

- 16 -

## TABLE I

### Antibody Production for Various 3GC Cell Lines at Passage 3

| Cell Line | Protein A Dectectable Human Gamma Globulin* (ng/ml) | Antitetanus Antibody ** (ng/ml) |
|---|---|---|
| 3GC-B1 | 3 | 0 |
| 3GC-B2 | 35 | 0 |
| 3GC-B3 | 23 | 0 |
| 3GC-B4 | 120 | 0 |
| 3GC-B5 | 5820 | 0 |
| 3GC-C1 | 3960 | 18 |
| 3GC-C2 | 1530 | 164 |
| 3GC-C3 | 2680 | 0 |
| 3GC-C4 | 209 | 14 |
| 3GC-C5 | 1600 | 19 |

\* Protein A probe does not detect subgroup 3 immunoglobulin G; values are averages of triplicate samples.

\*\* Values of antitetanus antibody production below 20 ng/ml were determined with triplicate undiluted and 10 X concentrated samples of media; zero indicates < 0.1 ng/ml.

0003173

## TABLE II

### Production of Antibody by Two Cell Lines

| Cell Line | Passage | Time in Culture (months) | Protein A Detectable Human Gamma Globulin + (ng/ml) | Antitetanus Antibody ++ (ng/ml) |
|---|---|---|---|---|
| 3GC-C2 | 0 | 1.0 | N.D. ‡ | 300 |
| | 1 | 1.5 | N.D. | 225 |
| | 2 | 1.7 | 1630 | 200 |
| | 3 | 2.1 | 1530 | 164 |
| | 5 | 2.5 | 2220 | 25 |
| | 6 | 3.1 | 1060 | 15* |
| | 9 | 4.0 | 1960 | 9* |
| | 15 | 4.2 | 1050 | 5* |
| | 24 | 5.3 | 930 | 2-3* |
| 4LP-B4 | 0 | 1.0 | N.D. | 150 |
| | 1 | 1.2 | N.D. | 105 |
| | 3 | 1.8 | N.D. | 75 |
| | 5 | 2.5 | N.D. | 15* |
| | 7 | 2.7 | 446 | 20* |
| | 8 | 2.8 | 515 | 15* |
| | 9 | 3.0 | 348 | 18* |
| | 16 | 4.0 | 272 | 8* |
| | 18 | 4.4 | 271 | 2* |
| | 21 | 4.8 | 242 | 2* |
| | 27 | 5.6 | 198 | 1* |
| | 40 | 7.2 | 259 | 0.1* |

+  Values represent averages of triplicate samples.

++ Values represent averages of 6 to 10 samples;  values below 25 ng/ml were determined with undiluted and 10 X concentrated media;  range of error about the mean for asterisked 3 GC-C2 data is 10-20% and for asterisked 4LP-B4 data is 10-50%.

‡  N.D. = not done.

## TABLE III

### Antitetanus Antibody Production by
### Subculture of Parent Line 3GC-C2

| Designation of 3GC-C2 Subculture | Passage of Parent Line at Time of Dilution | Initial Number of Cells in Subculture | Antitetanus Antibody + (ng/ml) | Antitetanus Antibody ++ (ng/ml) |
|---|---|---|---|---|
| 1D2 | 10 | 16000 | 54 | 103 |
| 1E3 | 10 | 1600 | 130 | 26 |
| 1F2 | 10 | 16000 | 101 | <1 |
| 1G3 | 10 | 16000 | 108 | 10-15 |
| 1H2 | 10 | 16000 | 227 | 10-15 |
| C5* | 10 | 10000 | 52 | 45 |
| 1D8 | 10 | 1600 | 177 | <1 |
| 1G5 | 10 | 1600 | 273 | 60 |
| 1H7 | 10 | 1600 | 156 | 25 |
| E10 | 10 | 1000 | 47 | 81 |
| F3 | 10 | 1000 | 178 | 648 |
| 2B1 | 13 | 9700 | 39 | <1 |
| 2D1 | 13 | 9700 | 95 | <1 |
| 2F2 | 13 | 9700 | 48 | 47 |
| 2G1 | 13 | 9700 | 51 | 60 |
| 2H8 | 13 | 970 | 456 | N.D.‡ |
| G11 | 23 | 17000 | 94 | <1 |
| D6 | 23 | 17000 | 38 | 22 |
| 3B10* | 23 | 1700 | 1550 | 37 |

+ Highest level of antibody achieved in microliter well in which dilution was accomplished.

++ Data obtained after two additional passages after limiting dilution subculture; cells initially in 0.1 ml have repopulated 2-3 ml to approximately $1 \times 10^6$/ml.

* These subcultures were not grown on feeder layers, all others were grown on human embryonic kidney monolayers.

‡ N.D. = not done.

- 1 -

0003173

<u>CLAIMS</u>

1.   A process for producing a human antibody which comprises

    a.   administering the antigen for the antibody to human lymphocyte cells to stimulate production of said antibody by said cells,

    b.   transforming said cells with a lymphotropic virus,

    c.   growing said transformed cells in vitro,

    d.   concentrating the transformed cells capable of producing said antibody specifically from other transformed cells that produce immunoglobulin without said antibody,

    e.   growing said concentrated transformed cells in a growth medium to form continuous cell lines, and

    f.   recovering said antibody from said continuous cell lines.

2.   A process according to claim 1 wherein said antigen is administered to said cells in vitro.

3.   A process according to claim 1 wherein said antigen is administered to said cells in vivo.

4.   A process according to any one of claims 1 to 3 wherein said virus is Epstein-Barr virus.

5.   A process according to any one of claims 1 to 4 wherein said cells are transformed in the presence of said antigen.

6.   A process according to any one of claims 1 to 5 wherein said antibody is antitetanus antibody.

7.   A process according to any one of claims 1 to 5 wherein said antibody is an antiviral antibody.

8.   A process according to any one of claims 1 to 5 wherein said antibody is anti-HLA.

9.   A process according to any one of claims 1 to 5 wherein said antibody is anti-tumor angionenesis antibody.

10. A process according to any one of claims 1 to 5 wherein the antibody is an antibody specific for a drug.

11. A process according to any one of claims 1 to 5 wherein the antibody is an antibody specific for a hormone.

12. A process according to any one of claims 1 to 5 wherein the antibody is an antibody specific for an antigenic determinant defining an organ, a tumor or a specific tissue.

13. A process according to any one of claims 1 to 12 wherein cells producing said antibody are concentrated from cells producing immunoglobulin without said antibody prior to administering said antigen.

14. A process according to any one of claims 1 to 13 wherein the antibody is directed against surface antigenic determinants of lymphocytes.

15. A process according to claim 14 wherein the surface antigenic determinant is that of the T cell.

16. A process according to claim 14 wherein the surface antigenic determinant is that of the B cell.

17. A process according to claim 14 wherein the surface antigenic determinant is that of the macrophage.

18. A composition comprising an antibody obtained by a process according to any one of claims 1 to 17 and a physiologically acceptable carrier.

European Patent Office

| DOCUMENTS CONSIDERED TO BE RELEVANT | | | CLASSIFICATION OF THE APPLICATION (Int. Cl.²) |
|---|---|---|---|
| Category | Citation of document with indication, where appropriate. of relevant passages | Relevant to claim | |
| | CHEMICAL ABSTRACTS, vol. 88, 1978 ref. 20304x Columbus, Ohio, U.S.A. K. NILSSON et al. "Surface glyco-protein patterns of normal and malignant human lymphoid cells. II. B cells, B blasts and Epstein-Barr virus (EBV)-positive and -negative B lymphoid cell lines" | 1,4 | A 61 K 39/00 C 12 K 9/00 A 61 K 39/40 39/42// G 01 N 33/16 |
| | ---- | | |

TECHNICAL FIELDS SEARCHED (Int.Cl.²)

A 61 K 39/00
39/40
39/42
C 12 K 5/00
7/00
9/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| The present search report has been drawn up for all claims | | |
|---|---|---|
| Place of search The Hague | Date of completion of the search 26-04-1979 | Examiner REMPP |

EPO Form 1503.1   06.78